# EUROPEAN PATENT APPLICATION

(11) **EP 4 371 590 A2**
(43) Date of publication of application: **22.05.2024**
(21) Application number: 23209302.1
(22) Date of filing: 13.11.2023
(51) Int. Cl.: A61M 1/36

(54) **MAXIMIZATION OF PLASMA COLLECTION AND COLLECTION OF BUFFY COAT OR WHITE BLOOD CELL LAYER USING COLLECTED PLASMA**

(30) Priority: 15.11.2022 US 202263425532 P
(71) Applicant: Fenwal, Inc., Lake Zurich, IL 60047 (US)
(72) Inventor: KUSTERS, Benjamin E., Lake Zurich, 60047 (US); BROWN, Richard I., Lake Zurich, 60047 (US); MIN, Kyungyoon, Lake Zurich, 60047 (US)
(74) Representative: Maikowski & Ninnemann Patentanwälte Partnerschaft mbB

(57) **Abstract**

A blood processing device includes a pump system, a valve system, a centrifuge, and a controller configured and/or programmed to control the operation of the pump system, the valve system, and the centrifuge to execute a blood separation procedure. The blood separation procedure executed by the controller includes pumping blood into the centrifuge, separating the blood in the centrifuge into red blood cells and plasma, and collecting portions of the separated red blood cells and plasma. Subsequently, a portion of the collected red blood cells is pumped back into the centrifuge to collect an additional amount of the separated plasma. A buffy coat or white blood cell layer may be formed between the separated red blood cells and plasma in the centrifuge. If so, a portion of the collected plasma may be pumped back into the centrifuge to harvest the buffy coat or white blood cell layer.

## Description

### Background

### Field of the Disclosure

The present disclosure relates to centrifugal separation of whole blood and collection of separated blood components. More particularly, the present disclosure relates to maximization of plasma collection and harvesting a buffy coat or white blood cell layer from a centrifuge chamber using collected plasma.

### Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

According to one approach, whole blood may be separated into its constituents through centrifugation. This requires that the whole blood be passed through a centrifuge after it is withdrawn from, and before it is returned to, the blood source. To reduce contamination and possible infection (if the blood source is a human donor or patient), the blood is preferably processed within a sealed, sterile fluid flow circuit during the centrifugation process. The operator installs a fresh, sterile disposable flow circuit in the centrifuge before processing and removes and discards it afterwards. Typical disposable flow circuits are sealed and sterile, and include a separation chamber portion, which is mounted in cooperation on a durable, reusable assembly containing the hardware (centrifuge, drive system, pumps, valve actuators, programmable controller, and the like) that rotates the separation chamber and controls the flow through the fluid circuit. The separation chamber may be formed of a generally rigid material (e.g., molded plastic), in which case the chamber itself defines a flow path or channel in which blood is separated into two or more components, or a more flexible material (e.g., in the form of a belt or annulus), which relies upon the system hardware to support the chamber and define the shape of the chamber as blood flows through it.

With a disposable circuit loaded onto the centrifuge (or just prior to or during loading) the operator typically enters, for example, by means of a touch screen or other user interface system, a particular processing protocol to be executed by the system (e.g., a procedure wherein platelets are separated from whole blood and collected) and other parameters (e.g., the weight of the donor, the desired volume of separated blood component to be collected, etc.). When the system has been programmed, the operator phlebotomizes a donor (or otherwise accesses the blood of the blood source) and the system carries out the procedure, under the supervision of the operator.

The centrifuge rotates the separation chamber of the disposable flow circuit during processing, causing the heavier (greater specific gravity) components of the whole blood in the separation chamber, such as red blood cells, to move radially outwardly away from the center of rotation toward the outer or "high-G" wall of the separation chamber. The lighter (lower specific gravity) components, such as plasma, migrate toward the inner or "low-G" wall of the separation chamber. The boundary that forms between the heavier and lighter components in the separation chamber is commonly referred to as the interface. Various ones of these components can be selectively removed from the whole blood by providing appropriately located channeling structures and outlet ports in the flow circuit. For example, in one blood separation procedure, blood is separated into plasma and red blood cells, with a buffy coat or white blood cell layer positioned therebetween. The buffy coat or white blood cell layer (which may include platelets and various white blood cells, such as mononuclear cells and possibly peripheral blood stem cells) is collected, with plasma and red blood cells being returned to the blood source or separately collected.

A system capable of executing such a red blood cell, plasma, and buffy coat collection procedure is described in PCT Patent Application Publication No. WO 2021/194824 A1, which is hereby incorporated herein by reference. More particularly, PCT Patent Application Publication No. WO 2021/194824 A1 describes a procedure in which blood in a continuous-flow centrifuge chamber is separated into plasma, buffy coat, and red blood cells. Separated plasma exits the chamber via a plasma outlet port and is collected in a plasma collection container, while separated red blood cells exit the chamber via a red blood cell outlet port and are collected in a red blood cell collection container. The buffy coat remains within the chamber while the other components are being collected.

During a buffy coat harvest stage, air from the plasma collection container is pumped into the chamber via the plasma outlet port. The air moves from the low-g wall of the chamber toward the high-g wall as additional air is conveyed into the chamber, which displaces the buffy coat remaining in the chamber through the red blood cell outlet port. The buffy coat is directed from the red blood cell outlet port into a buffy coat collection container, where it is collected as a buffy coat product.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood processing device includes a pump system, a centrifuge, and a controller. The controller is configured to actuate the pump system to convey blood from a blood source into the centrifuge and then actuate the centrifuge to separate the blood in the centrifuge into plasma, red blood cells, and a buffy coat or white blood cell layer between the separated plasma and the separated red blood cells. The controller actuates the pump system to convey the separated plasma and the separated red blood cells out of the centrifuge for collection and then actuates the pump system to convey a portion of the collected plasma into the centrifuge to convey the buffy coat or white blood cell layer out of the centrifuge for collection.

In another aspect, a blood processing system includes a reusable processing device and a disposable fluid flow circuit. The processing device has a pump system, a centrifuge, and a controller, while the fluid flow circuit has a processing chamber received by the centrifuge, a buffy coat or white blood cell layer collection container, and a plurality of conduits fluidly connecting the components of the fluid flow circuit. The controller is configured to actuate the pump system to convey blood from a blood source into the processing chamber and then actuate the centrifuge to separate the blood in the processing chamber into plasma, red blood cells, and a buffy coat or white blood cell layer between the separated plasma and the separated red blood cells. The controller actuates the pump system to convey the separated plasma and the separated red blood cells out of the processing chamber for collection and then actuates the pump system to convey a portion of the collected plasma into the processing chamber to convey the buffy coat or white blood cell layer out of the processing chamber and into the buffy coat or white blood cell collection container.

In yet another aspect, a method is provided for collecting a buffy coat or white blood cell product. The method includes conveying blood from a blood source into a centrifuge and then separating the blood in the centrifuge into plasma, red blood cells, and a buffy coat or white blood cell layer between the separated plasma and the separated red blood cells. The separated plasma and the separated red blood cells are conveyed out of the centrifuge for collection and then a portion of the collected plasma is conveyed into the centrifuge to convey the buffy coat or white blood cell layer out of the centrifuge for collection.

In another aspect, a blood processing device includes a pump system, a centrifuge, and a controller. The controller is configured to actuate the pump system to convey blood from a blood source into the centrifuge and then actuate the centrifuge to separate the blood in the centrifuge into separated plasma and separated red blood cells. The controller actuates the pump system to simultaneously convey a first portion of the separated plasma out the centrifuge and into a plasma collection container, while conveying a first portion of the separated red blood cells out of the centrifuge and into a temporary storage container. The controller then actuates the pump system to convey the separated red blood cells out of the temporary storage container and into the centrifuge to convey an additional portion of the separated plasma into the plasma collection container.

In yet another aspect a blood processing system includes a reusable processing device and a disposable fluid flow circuit. The processing device includes a pump system, a centrifuge, and a controller, while the fluid flow circuit includes a processing chamber received by the centrifuge, a temporary storage container, a plasma collection container, and a plurality of conduits fluidly connecting the components of the fluid flow circuit. The controller is configured to actuate the pump system to convey blood from a blood source into the processing chamber and then actuate the centrifuge to separate the blood in the processing chamber into separated plasma and separated red blood cells. The controller actuates the pump system to simultaneously convey a first portion of the separated plasma out the processing chamber and into the plasma collection container, while conveying a first portion of the separated red blood cells out of the centrifuge and into the temporary storage container. The controller then actuates the pump system to convey the separated red blood cells out of the temporary storage container and into the processing chamber to convey an additional portion of the separated plasma into the plasma collection container.

In another aspect, a method is provided for collecting plasma. The method includes conveying blood from a blood source into a centrifuge and then separating the blood in the centrifuge into separated plasma and separated red blood cells. A first portion of the separated plasma is conveyed out the centrifuge and into a plasma collection container while a first portion of the separated red blood cells is simultaneously conveyed out of the centrifuge and into a temporary storage container. The separated red blood cells are then conveyed out of the temporary storage container and into the centrifuge to convey an additional portion of the separated plasma into the plasma collection container.

### Brief Description of the Drawings

Fig. 1 is a perspective view of an exemplary reusable hardware component of a blood processing system which is configured to receive a disposable fluid flow circuit;
Fig. 2 is a plan view of an exemplary disposable fluid flow circuit for use in combination with the durable hardware component of Fig. 1;
Fig. 3 is a schematic view of the fluid flow circuit of Fig. 2 mounted to the processing device of Fig. 1 to complete a blood processing system according to an aspect of the present disclosure;
Fig. 4 is a schematic view of the blood processing system of Fig. 3 executing a "blood prime" stage of an exemplary blood processing procedure;
Fig. 5 is a schematic view of the blood processing system of Fig. 3 executing an "establish separation" stage of an exemplary blood processing procedure;
Fig. 6 is a schematic view of the blood processing system of Fig. 3 executing a "red blood cell divert" stage of an exemplary blood processing procedure;
Fig. 7 is a schematic view of the blood processing system of Fig. 3 executing a "collection" stage of an exemplary blood processing procedure;
Fig. 8 is a schematic view of the blood processing system of Fig. 3 executing a "plasma maximization" stage of an exemplary blood processing procedure;
Fig. 9 is a schematic view of the blood processing system of Fig. 3 executing a "red blood cell recovery" stage of an exemplary blood processing procedure;
Fig. 10 is a schematic view of the blood processing system of Fig. 3 executing a "buffy coat harvest" stage of an exemplary blood processing procedure;
Fig. 11 is a schematic view of the blood processing system of Fig. 3 executing an "additive solution flush" stage of an exemplary blood processing procedure;
Fig. 12 is a schematic view of the blood processing system of Fig. 3 executing an "air evacuation" stage of an exemplary blood processing procedure; and
Fig. 13 is a schematic view of the blood processing system of Fig. 3 executing a "sealing" stage of an exemplary blood processing procedure.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

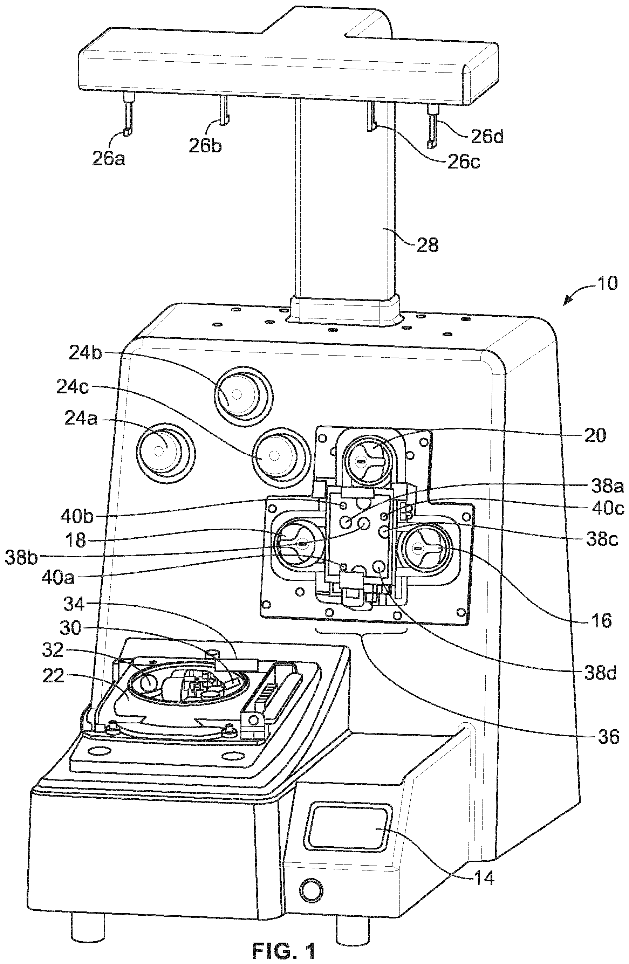

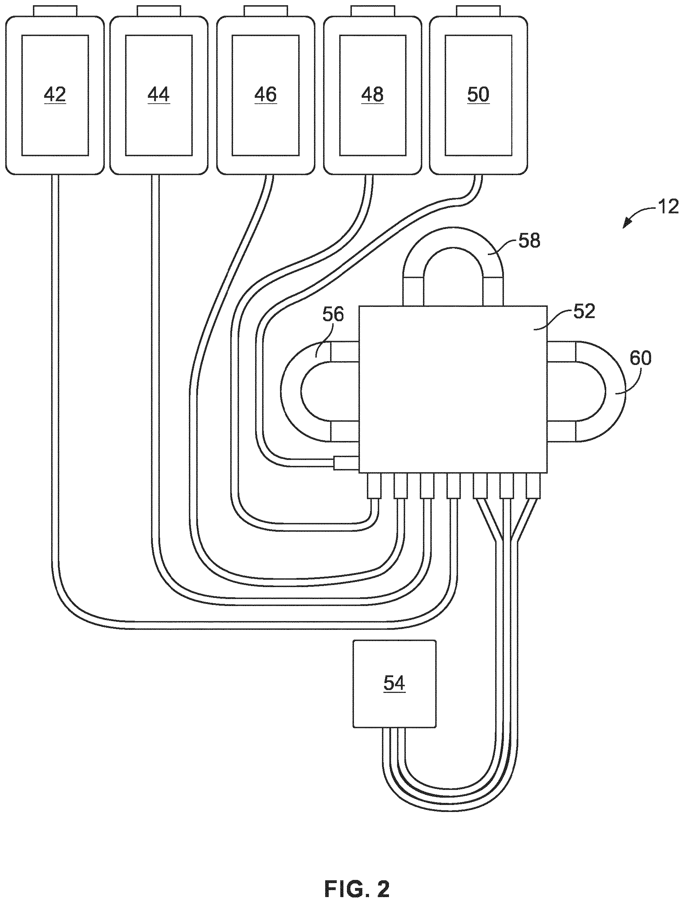

Fig. 1 depicts a reusable hardware component or processing device of a blood processing system, generally designated 10, while Fig. 2 depicts a disposable fluid flow circuit, generally designated 12, to be used in combination with the processing device 10 for processing blood. The illustrated processing device 10 includes associated pumps, valves, sensors, displays, and other apparatus for configuring and controlling flow of fluid through the fluid flow circuit 12, described in greater detail below. The blood processing system may be directed by a controller integral with the processing device 10 that includes a programmable microprocessor to automatically control the operation of the pumps, valves, sensors, etc. The processing device 10 may also include wireless communication capabilities to enable the transfer of data from the processing device 10 to the quality management systems of the operator.

More specifically, the illustrated processing device 10 includes a user input and output touchscreen 14, a pump station including a first pump 16 (for pumping, e.g., whole blood), a second pump 18 (for pumping, e.g., plasma) and a third pump 20 (for pumping, e.g., additive solution), a centrifuge mounting station and drive unit 22 (which may be referred to herein as a "centrifuge"), and clamps 24a-c. The touchscreen 14 enables user interaction with the processing device 10, as well as the monitoring of procedure parameters, such as flow rates, container weights, pressures, etc. The pumps 16, 18, and 20 (collectively referred to herein as being part of a "pump system" of the processing device 10) are illustrated as peristaltic pumps capable of receiving tubing or conduits and moving fluid at various rates through the associated conduit dependent upon the procedure being performed. An exemplary centrifuge mounting station/drive unit is seen in U.S. Patent No. 8,075,468 (with reference to Figs. 26-28), which is hereby incorporated herein by reference. The clamps 24a-c (collectively referred to herein as being part of the "valve system" of the processing device 10) are capable of opening and closing fluid paths through the tubing or conduits and may incorporate RF sealers in order to complete a heat seal of the tubing or conduit placed in the clamp to seal the tubing or conduit leading to a product container upon completion of a procedure.

Sterile connection/docking devices may also be incorporated into one or more of the clamps 24a-c. The sterile connection devices may employ any of several different operating principles. For example, known sterile connection devices and systems include radiant energy systems that melt facing membranes of fluid flow conduits, as in U.S. Patent No. 4,157,723; heated wafer systems that employ wafers for cutting and heat bonding or splicing tubing segments together while the ends remain molten or semi-molten, such as in U.S. Patent Nos. 4,753,697; 5,158,630; and 5,156,701; and systems employing removable closure films or webs sealed to the ends of tubing segments as described, for example, in U.S. Patent No. 10,307,582. Alternatively, sterile connections may be formed by compressing or pinching a sealed tubing segment, heating and severing the sealed end, and joining the tubing to a similarly treated tubing segment as in, for example, U.S. Patent Nos. 10,040,247 and 9,440,396. All of the above-identified patents are incorporated by reference in their entirety. Sterile connection devices based on other operating principles may also be employed without departing from the scope of the present disclosure.

The processing device 10 also includes hangers 26a-d (which may each be associated with a weight scale) for suspending the various containers of the disposable fluid circuit 12. The hangers 26a-d are preferably mounted to a support 28, which is vertically translatable to improve the transportability of the processing device 10. An optical system comprising a laser 30 and a photodetector 32 is associated with the centrifuge 22 for determining and controlling the location of an interface between separated blood components within the centrifuge 22. An exemplary optical system is shown in U.S. Patent Application Publication No. 2019/0201916, which is hereby incorporated herein by reference. An optical sensor 34 is also provided to optically monitor one or more conduits leading into or out of the centrifuge 22.

The face of the processing device 10 includes a nesting module 36 for seating a flow control cassette 52 (Fig. 2) of the fluid flow circuit 12. The cassette nesting module 36 is configured to receive various disposable cassette designs so that the system may be used to perform different types of procedures. Embedded within the illustrated cassette nesting module 36 are four valves 38a-d (collectively referred to herein as being part of the "valve system" of the processing device 10) for opening and closing fluid flow paths within the flow control cassette 52, and three pressure sensors 40a-c capable of measuring the pressure at various locations of the fluid flow circuit 12.

With reference to Fig. 2, the illustrated fluid flow circuit 12 includes a plurality of containers 42, 44, 46, 48, and 50 with a flow control cassette 52 and a processing/separation chamber 54 that is configured to be received in the centrifuge 22, all of which are interconnected by conduits or tubing segments, so as to permit continuous flow centrifugation. The flow control cassette 52 routes the fluid flow through three tubing loops 56, 58, 60, with each loop being positioned to engage a particular one of the pumps 16, 18, 20. The conduits or tubing may extend through the cassette 52, or the cassette 52 may have pre-formed fluid flow paths that direct the fluid flow.

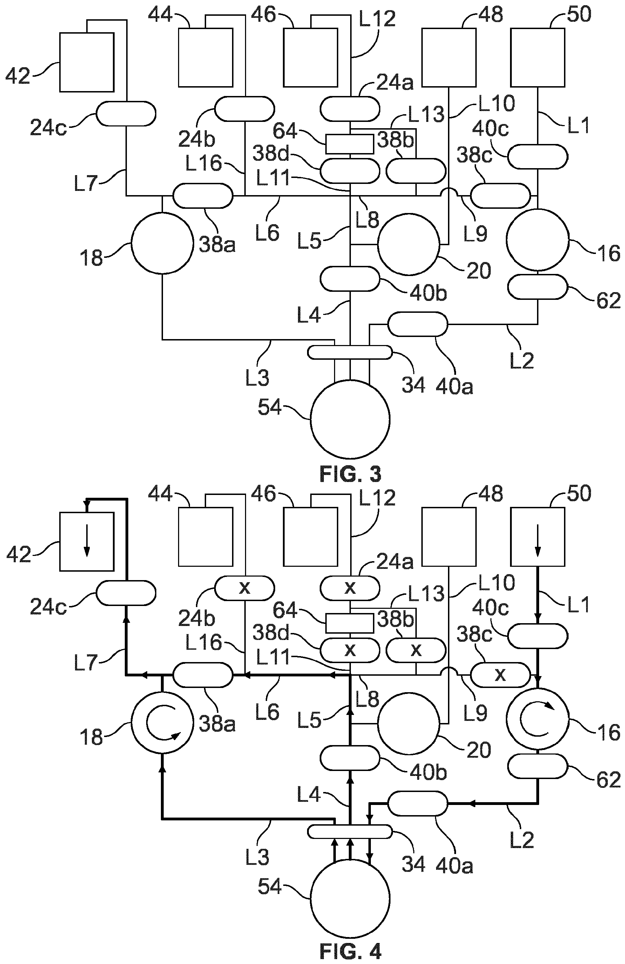

In the fluid flow circuit 12 shown in Fig. 2, containers 42, 44, and 46 may be empty containers for the receipt of plasma, a buffy coat or white blood cell layer, and red blood cells (respectively) separated from whole blood, container 48 may be prefilled with additive solution, and container 50 may be filled with whole blood and connected to the fluid flow circuit 12 at the time of use. While Fig. 2 shows a whole blood container 50 (configured as a blood pack unit, for example) as a blood source, it is within the scope of the present disclosure for the blood source to be a living donor. The fluid flow circuit 12 may optionally include an air trap 62 (Fig. 3) through which the whole blood is flowed prior to entering the separation chamber and/or a leukoreduction filter 64 through which the red blood cells are flowed prior to entering the red blood cell collection container 46.

The processing chamber 54 may be pre-formed in a desired shape and configuration by injection molding from a rigid plastic material, as shown and described in U.S. Patent No. 6,849,039, which is hereby incorporated herein by reference. The specific geometry of the processing chamber 54 may vary depending on the elements to be separated, and the present disclosure is not limited to the use of any specific chamber design. For example, it is within the scope of the present disclosure for the processing chamber 54 to be configured formed of a generally flexible material, rather than a generally rigid material. When the processing chamber 54 is formed of a generally flexible material, it relies upon the centrifuge 22 to define a shape of the processing chamber 54. An exemplary processing chamber formed of a flexible material and an associated centrifuge are described in U.S. Patent No. 6,899,666, which is hereby incorporated herein by reference.

The controller of the processing device 10 may be pre-programmed to automatically operate the system to perform one or more standard blood processing procedures selected by an operator by input to the touchscreen 14, and configured to be further programmed by the operator to perform additional blood processing procedures. The controller may be pre-programmed to substantially automate a wide variety of procedures, including, but not limited to: red blood cell and plasma production from a single unit of whole blood, buffy coat pooling, buffy coat separation into a platelet product (as described in U.S. Patent Application Publication No. 2018/0078582, which is hereby incorporated herein by reference), glycerol addition to red blood cells, red blood cell washing, platelet washing, and cryoprecipitate pooling and separation.

The pre-programmed blood processing procedures operate the system at pre-set settings for flow rates and centrifugation forces, and the programmable controller may be further configured to receive input from the operator as to one or more of flow rates and centrifugation forces for the standard blood processing procedure to override the pre-programmed settings. In addition, the programmable controller may be configured to receive input from the operator through the touchscreen 14 for operating the system to perform a non-standard blood processing procedure.

According to an aspect of the present disclosure, the processing device 10 and the fluid flow circuit 12 may be used in combination to process a volume of whole blood into a red blood cell product, a plasma product, and a buffy coat or white blood cell product. Fig. 3 is a schematic illustration of the fluid flow circuit 12 mounted to the processing device 10, with selected components of the fluid flow circuit 12 and selected components of the processing device 10 being shown. Figs. 4-13 show different stages of an exemplary procedure. In the procedure to be described, plasma is separated from cellular blood components as platelet-poor plasma, with platelets separated from the plasma settling into the buffy coat layer (along with assorted white blood cells). In other embodiments, a separated plasma layer may comprise platelet-rich plasma, in which case there is an intermediate layer of white blood cells (which may be predominantly mononuclear cells and peripheral blood stem cells) between the plasma layer and the red blood cell layer. In either case, the procedure is similar, such that references herein to "plasma" or a "plasma layer" are intended to encompass both platelet-poor plasma and platelet-rich plasma, while the intermediate blood component layer (between the plasma layer and the red blood cell layer) will be referred to as the buffy coat or white blood cell layer (with it being understood that a buffy coat accompanies a platelet-poor plasma layer while a white blood cell layer accompanies a platelet-rich plasma layer).

In an initial stage, selected components of the fluid flow circuit 12 are primed to remove air therefrom. While it is within the scope of the present disclosure for a separately provided fluid (e.g., anticoagulant or saline) to be used for priming the fluid flow circuit 12, Fig. 4 illustrates a "blood prime" stage in which regions of the fluid flow circuit 12 are primed using blood from a blood source. The blood source is shown in Fig. 4 as the whole blood container 50, but may alternatively be a living donor. Thus, it should be understood that the term "whole blood" may refer to blood that either includes or omits an anticoagulant fluid.

During the blood prime stage, whole blood is drawn into the fluid flow circuit 12 from the blood source (the whole blood container 50 in the embodiment of Fig. 4) via line L1 by operation of the first pump 16 (which may be referred to as the "whole blood pump"). Valve 38c is closed, which directs the blood through pressure sensor 40c and into line L2. The blood passes through air trap 62, pressure sensor 40a (which measures the pressure of the processing chamber 54), and optical sensor 34 before flowing into the processing chamber 54, which is positioned within the centrifuge 22 of the processing device 10.

The centrifuge 22 may be stationary during the blood prime stage or may instead be controlled by the controller of the processing device 10 to spin at a low rotation rate (e.g., on the order of approximately 1,000-2,000 rpm). It may be advantageous for the centrifuge 22 to rotate during the blood prime stage in order to create enough g-force to ensure that the air in the processing chamber 54 (which includes air already present in the processing chamber 54, along with air moved into the processing chamber 54 from lines L1 and/or L2 by the flow of blood) is forced towards the low-G (radially inner) wall of the processing chamber 54. Higher centrifuge rotation rates, such as 4,500 rpm (which is required for steady state separation, as will be described) may be undesirable as air blocks (in which air gets stuck and cannot be forced out of the processing chamber 54, causing pressure to rise) are more likely at higher g-forces.

The blood entering the processing chamber 54 will move towards the high-G (radially outer) wall of the processing chamber 54, displacing air towards the low-G wall. A plasma outlet port of the processing chamber 54 is associated with the low-G wall of the processing chamber 54, such that most of the air will exit the processing chamber 54 via the plasma outlet port and associated line L3, although some air may also exit processing chamber 54 via a red blood cell outlet port associated with the high-G wall of the processing chamber 54.

Valves 38b and 38d and clamp 24b are closed, while the second pump 18 (which may be referred to as the "plasma pump") is active and the third pump 20 (which may be referred to as the "additive pump") is inactive. Such an arrangement will direct the air exiting the processing chamber 54 via the red blood cell outlet port through associated line L4 and pressure sensor 40b, into line L5 and then into line L6. Valve 38a is open, such that the air flowing through line L6 will meet up with the air flowing through line L3 (i.e., the air that exits the processing chamber 54 via the plasma outlet port). The combined air will flow through line L7 and open clamp 24c, into the plasma collection container 42. It should be understood that, in Figs. 4-13, arrows on the containers represent the direction of fluid flow between the container and the conduit connected to the container. For example, line L7 is shown as being connected to the top of the plasma collection container 42, such that a downward arrow (as in Fig. 4) represents downward fluid flow into the plasma collection container 42. In contrast, line L1 is shown as being connected to the bottom of the whole blood container 50, such that a downward arrow (as in Fig. 4) represents downward fluid flow out of the whole blood container 50.

The flow of air out of the processing chamber 54 via either outlet port is monitored by the optical sensor 34, which is capable of determining the optical density of the fluid flowing through the monitored lines and discerning between air and a non-air fluid in lines L3 and L4. When a non-air fluid is detected in both lines L3 and L4, the controller of the processing device 10 will end the blood prime stage and move on to the next stage of the procedure. The amount of blood drawn into the fluid flow circuit 12 from the blood source during the blood prime stage will vary depending on a number of factors (e.g., the amount of air in the fluid flow circuit 12), but may be on the order of approximately 50 to 100 mL. The blood prime stage may take on the order of one to two minutes.

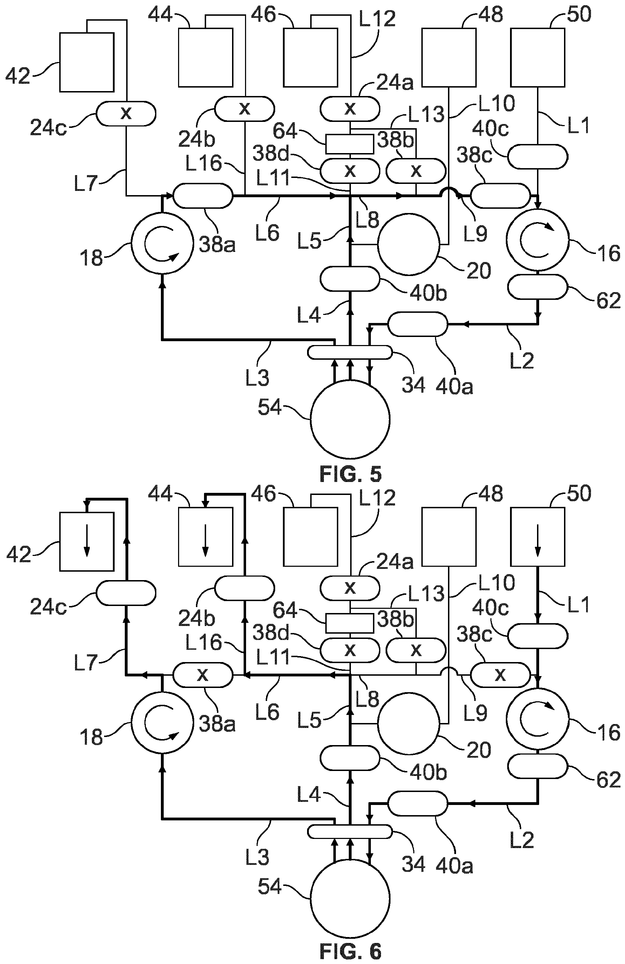

Fig. 5 shows an optional next stage that is referred to herein as the "establish separation" stage. This stage may be advantageous when the amount of blood to be separated is limited, such as when the blood source is a blood pack containing one unit of blood. In other embodiments in which the supply of blood is not so limited, the establish separation stage may be omitted or modified or may proceed as described below.

When an establish separation stage is to be executed, it begins once non-air fluid has been detected in lines L3 and L4. At this time, the rotational speed of the centrifuge 22 will be increased to a rate that is sufficient to separate blood into packed red blood cells and plasma (which may be in the range of approximately 4,500 to 5,500 rpm, in the case of separation of platelet-poor plasma from cellular blood components). If it is desired to produce a plasma product that is low in platelets, the processing chamber 54 may be configured with a plasma outlet port that is spaced from and positioned downstream of the blood inlet port, rather than being positioned adjacent to the blood inlet port. Such a configuration allows the platelets to settle down into the buffy coat before the plasma is removed from the processing chamber 54, thus allowing the separated plasma to be platelet-depleted. A differently configured processing chamber 54 (and a different, lower rotational speed) may be employed if a platelet-rich plasma layer is to be separated from the blood. As for the whole blood pump 16, it continues to operate, but no additional blood is drawn into the fluid flow circuit 12 from the blood source during the establish separation stage.

When the supply of blood is limited and the system must work with a finite fluid volume, the establish separation stage is advantageous to avoid product loss or quality issues. These goals are achieved by the separated plasma and red blood cells being removed from the processing chamber 54, mixed together to form recombined whole blood, and recirculated back into the processing chamber 54, rather than the separated components being directed to their respective collection containers.

More particularly, during the establish separation stage, separated plasma will exit the processing chamber 54 via the plasma outlet port and associated line L3. Clamp 24c is closed during this stage, while valve 38a remains open, which directs the plasma from line L3 into line L6. Separated red blood cells exit the processing chamber 54 via the red blood cell outlet port and associated line L4. In the illustrated embodiment, there is no pump associated with line L4, such that the red blood cells exit the processing chamber 54 at a rate that is equal to the difference between the rate of the whole blood pump 16 and the rate of the plasma pump 18. In alternative embodiments, there may be a pump associated with the red blood cell outlet line instead of the plasma outlet line or a first pump associated with the plasma outlet line and a second pump associated with the red blood cell outlet line.

The additive pump 20 is inactive during this stage, thereby directing the red blood cells from line L4 into line L5. The plasma flowing through line L6 is mixed with the red blood cells flowing through line L5 at a junction of the two lines L5 and L6 to form recombined whole blood. Valve 38d is closed, which directs the recombined whole blood into line L8. Valve 38b is also closed, which directs the recombined whole blood from line L8 into line L9 and through open valve 38c. The whole blood pump 16 draws the recombined whole blood into line L2 from line L9 (rather than drawing additional blood into the fluid flow circuit 12 from the blood source), with the recombined blood passing through air trap 62, pressure sensor 40a, and optical sensor 34 before flowing back into the processing chamber 54, where it is again separated into plasma and red blood cells (with buffy coat therebetween).

The establish separation stage continues until steady state separation has been achieved, which may take on the order of approximately one to two minutes. As used herein, the phrase "steady state separation" refers to a state in which blood is separated into its constituents in the processing chamber 54, with the radial position of the interface between separated components within the processing chamber 54 being at least substantially maintained (rather than moving radially inwardly or outwardly). The position of the interface may be determined and controlled according to any suitable approach, including using an interface detector of the type described in U.S. Patent Application Publication No. 2019/0201916.

Preferably, steady state separation is achieved with the interface between separated components within the processing chamber 54 at a target location. The target location may correspond to the location of the interface at which separation efficiency is optimized, with the precise location varying depending on a number of factors (e.g., the hematocrit of the whole blood). However, in an exemplary embodiment, the target location of the interface may be the position of the interface when approximately 52% of the thickness or width (in a radial direction) of the channel defined by the processing chamber 54 is occupied by red blood cells. In the illustrated embodiment, the position of the interface within the processing chamber 54 may be adjusted by changing the flow rate of the plasma pump 18, with the flow rate being increased to draw more separated plasma out of the processing chamber 54 (which decreases the thickness of the plasma layer within the processing chamber 54) and move the interface toward the low-G wall or decreased to draw less plasma out of the processing chamber 54 (which increases the thickness of the plasma layer within the processing chamber 54) and move the interface toward the high-G wall.

In an exemplary procedure, the controller of the processing device 10 will control the whole blood pump 16 to operate at a constant rate, with the plasma pump 18 initially operating at the same rate, which will quickly increase the thickness of the red blood cell layer within the processing chamber 54 and move the interface toward the low-G wall. The rate of the plasma pump 18 is gradually decreased as the thickness of the red blood cell layer increases and the location of the interface approaches the target location. As described above, the target location of the interface may depend upon the hematocrit of the whole blood, meaning that the rate of the plasma pump 18 (which controls the position of the interface) may also depend on the hematocrit of the whole blood. In one embodiment, this relationship may be expressed as follows:

Theoretical plasma pump rate = whole blood pump rate - ((whole blood hematocrit * whole blood pump rate) / hematocrit of separated red blood cells) [Equation 1]

The hematocrit of the whole blood may be measured before the procedure begins or by the optical sensor 34 during the procedure, while the hematocrit of the separated red blood cells may be determined during the procedure by the optical sensor 34 monitoring line L4. In practice, the plasma pump rate will typically not remain at the theoretical rate once steady state separation has been achieved, with the interface at the target location, but rather the plasma pump rate will instead tend to "flutter" around the theoretical rate.

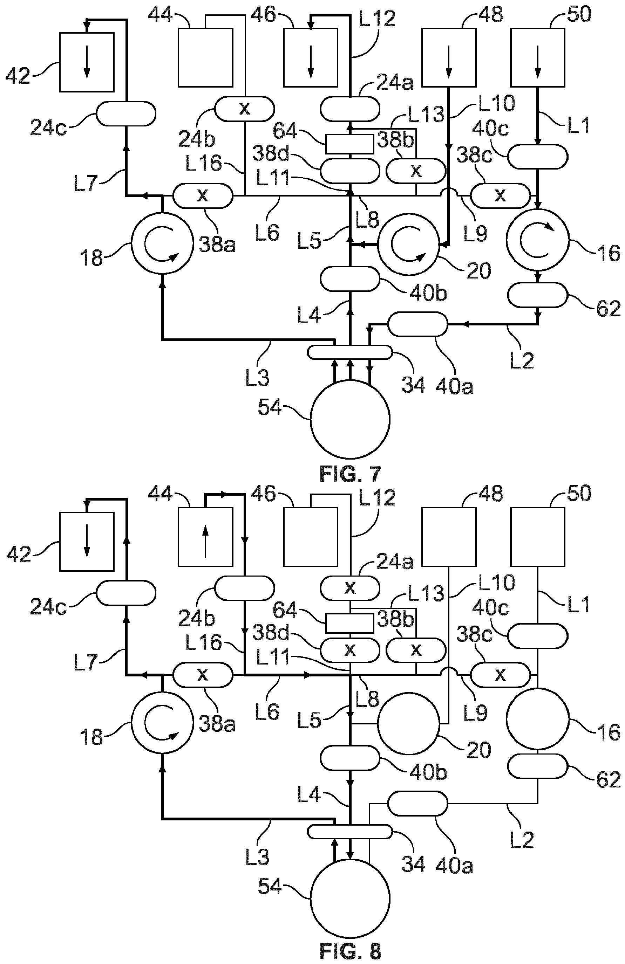

Regardless of the particular manner in which the controller of the processing device 10 executes the establish separation stage (if such a stage is executed at all), once steady state separation has been established, the controller advances the procedure to either an optional "red blood cell divert" stage (Fig. 6) or a "collection" stage (Fig. 7).

At the beginning of the red blood cell divert stage, the centrifuge 22, the whole blood pump 16, and the plasma pump 18 all continue operating at the same rates at which they were operating at the end of the establish separation stage (if implemented). The valve system of the processing device 10, however, is adjusted to direct the separated plasma to the plasma collection container 42 and to direct the separated red blood cells to the buffy coat or white blood cell layer collection container 44 (which serves as a temporary storage container for this first portion of the separated red blood cells). As in the establish separation stage, the buffy coat or white blood cell layer remains in the processing chamber 54 during the red blood cell divert stage.

Later (during a stage that is referred to herein as the "plasma maximization" stage and shown in Fig. 8), the red blood cells in the buffy coat or white blood cell layer collection container 44 are used to collect an additional amount of plasma in the plasma collection container 42, as will be described in greater detail. Thus, if maximization of the amount of plasma that is collected is not an objective of the procedure, the red blood cell divert stage (and the subsequent plasma maximization stage) may be omitted. However, collecting an increased volume of separated plasma has both therapeutic and financial advantages, such that those who process whole blood may prefer to execute both of the red blood cell divert and plasma maximization stages.

More particularly, during the red blood cell divert stage, valve 38c is closed, which causes the whole blood pump 16 to draw additional blood into line L1 from the blood source. The whole blood pump 16 draws the blood from the blood source into line L2 from line L1, with the blood passing through air trap 62, pressure sensor 40a, and optical sensor 34 before flowing into the processing chamber 54, where it is separated into plasma, red blood cells, and a buffy coat or white blood cell layer.

The separated plasma exits the processing chamber 54 via the plasma outlet port and associated line L3. Valve 38a is closed, which directs the plasma from line L3 into line L7, through open clamp 24c, and into the plasma collection container 42 (as a first portion of collected plasma).

The separated red blood cells exit the processing chamber 54 via the red blood cell outlet port and associated line L4. The additive pump 20 is inactive during this stage (as it may be desirable for the red blood cells collected during this stage to omit additive fluid), along with valves 38b and 38d being closed, which directs the red blood cells into and through line L5 and into line L6. As valve 38a is closed, the red blood cells are directed from line L6 into line L16, with the red blood cells passing through open clamp 24b and into the buffy coat or white blood cell layer collection container 44. While Fig. 6 illustrates the buffy coat or white blood cell layer collection container 44 serving as a temporary storage container for the separated red blood cells during this stage, it should be understood that some other container (which may preferably be empty) may serve as a temporary storage container.

The red blood cell divert stage continues until a target volume of red blood cells has been conveyed into the buffy coat or white blood cell layer collection container 44. The exact volume of red blood cells collected during this stage may vary without departing from the scope of the present disclosure, although it may be advantageous for the volume of red blood cells collected to correspond to or be based at least in part on the volume of plasma to be collected using the red blood cells during the plasma maximization stage. Similarly, the manner in which the amount of collected red blood cells is measured may also vary without departing from the scope of the present disclosure. For example, a weight scale supporting or otherwise associated with the buffy coat or white blood cell layer collection container 44 may be used to determine the weight (and, hence, the volume) of red blood cells present in the container 44. Alternatively, the rates at which the various pumps of the pump system are operating may be used to determine the volume of red blood cells that has been conveyed from the processing chamber 54 into the buffy coat or white blood cell layer collection container 44.

Once the target volume of red blood cells has been conveyed into the buffy coat or white blood cell layer collection container, the controller may transition the procedure to the collection stage of Fig. 7. At the beginning of the collection stage, the centrifuge 22, the whole blood pump 16, and the plasma pump 18 all continue operating at the same rates at which they were operating at the end of the previous stage (which may be the establish separation stage of Fig. 5 or the red blood cell divert stage of Fig. 6, depending on the particular procedure). The valve system of the processing device 10, however, is adjusted to direct the separated plasma and red blood cells to their respective collection containers, rather than recombining them and recirculating them through the centrifuge 22 (as in the establish separation stage) or diverting the separated red blood cells into a temporary storage container (as in the red blood cell divert stage). Additional blood is drawn into the fluid flow circuit 12 from the blood source until a target volume of whole blood (which may be a total of one unit of blood, in one embodiment) has been drawn into the fluid flow circuit 12. As in the establish separation and red blood cell divert stages, the buffy coat or white blood cell layer remains in the processing chamber 54 during the collection stage.

More particularly, during the collection stage, valve 38c is closed (as in the red blood cell divert stage), which causes the whole blood pump 16 to draw additional blood into line L1 from the blood source. The whole blood pump 16 draws the blood from the blood source into line L2 from line L1, with the blood passing through air trap 62, pressure sensor 40a, and optical sensor 34 before flowing into the processing chamber 54, where it is separated into plasma, red blood cells, and a buffy coat or white blood cell layer.

The separated plasma exits the processing chamber 54 via the plasma outlet port and associated line L3. Valve 38a is closed (as in the red blood cell divert stage), which directs the plasma from line L3 into line L7, through open clamp 24c, and into the plasma collection container 42 (as a second portion of collected plasma).

The separated red blood cells exit the processing chamber 54 via the red blood cell outlet port and associated line L4. The additive pump 20 is operated by the controller to draw an additive solution (which may be ADSOL^{®} or some other red blood cell additive) from the additive solution container 48 via line L10. The red blood cells flowing through line L4 are mixed with the additive solution flowing through line L10 at a junction of the two lines L4 and L10 to form a mixture that continues flowing into and through line L5. The mixture is ultimately directed into the red blood cell collection container 46, but may first be conveyed into line L11 and through a leukoreduction filter 64 (as shown in Fig. 7) before flowing through line L12 and open clamp 24a, into the red blood cell collection container 46 (as a first volume of collected red blood cells). Alternatively, the valve system may be controlled to cause the mixture to bypass the leukoreduction filter 64 (via line L13) and enter the red blood cell collection container 46 without being leukoreduced. It is also within the scope of the present disclosure for the mixture to be routed through the leukoreduction filter 64 at the beginning of the collection stage, with the valve system being reconfigured during the collection stage to cause the mixture to bypass the leukoreduction filter 64, such that only a portion of the collected red blood cells are leukoreduced. In one embodiment, pressure sensor 40b monitors the pressure of the leukoreduction filter 64. If the pressure sensor 40b detects that the pressure of the leukoreduction filter 64 has risen above a predetermined pressure threshold (which may be indicative of filter blockage), the controller may reconfigure the valve system to cause the mixture to bypass the leukoreduction filter 64. The system may then alert the operator that the red blood cell product was not leukoreduced.

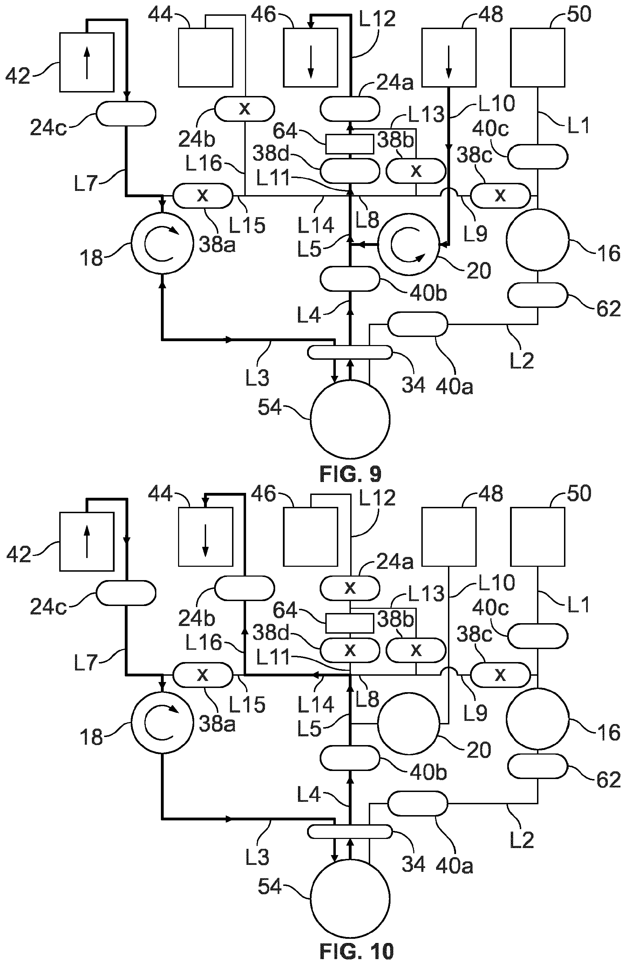

The collection stage continues until the target volume of whole blood has been drawn into the fluid flow circuit 12 from the blood source (which may be determined by any suitable approach). Once the target volume of whole blood has been drawn into the fluid flow circuit, the controller may transition the procedure to the optional plasma maximization stage (Fig. 7), an optional "red blood cell recovery" stage (Fig. 9), or a buffy coat harvest stage (Fig. 10). As noted above, the plasma maximization stage is only implemented when the red blood cell divert stage (Fig. 6) has been executed, with a volume of separated red blood cells in the buffy coat or white blood cell layer collection container 44.

During the plasma maximization stage, the red blood cells in the buffy coat or white blood cell layer collection container 44 (or in some other temporary storage container) are used to collect an additional amount (or third portion) of separated plasma. In the illustrated embodiment, the whole blood pump 16 and the additive pump 20 are deactivated, with valves 38b and 38d being closed (if not already closed) and clamp 24b being opened. With only the plasma pump 18 operational, it will draw the red blood cells out of the buffy coat or white blood cell layer collection container 44 via line L16. The red blood cells flow through open clamp 24b and into and through lines L6 and L5. From line L5, the red blood cells flow into and through line L4, entering the processing chamber 54 via the red blood cell outlet port.

On account of the red blood cells flowing through the red blood cell outlet port, they will enter the processing chamber 54 at the high-g side. As additional red blood cells are introduced into the processing chamber 54, they will move from the high-g wall towards the low-g wall, thus displacing the separated plasma in the processing chamber 54 through the plasma outlet port at the low-g side and into line L3. The plasma flows through line L3, into line L7, through open clamp 24c, and into the plasma collection container 42 (as a third portion of collected plasma).

As described above, the red blood cells in the buffy coat or white blood cell layer collection container 44 preferably do not include an additive solution. If an additive solution were pumped back into the processing chamber 54 (along with the red blood cells) during the plasma maximization stage, it could separate from the red blood cells in the processing chamber 54 and end up in the plasma collection container 42 at the end of the procedure. As the presence of additive solution in the plasma collection container 42 would decrease the purity of the collected plasma, it is advantageous for this portion of the plasma to be collected during this stage using red blood cells free of additive solution (which is why the red blood cells in the red blood cell collection container 46 are not used to collect plasma during this stage).

The plasma maximization stage continues until the separated plasma has been removed from the processing chamber 54. This may be determined in any of a number of ways without departing from the scope of the present disclosure. In one embodiment, the plasma maximization stage continues until a predetermined volume of fluid (corresponding to the volume of red blood cells pumped into the processing chamber 54 from the buffy coat or white blood cell layer collection container 44) has been conveyed out of the processing chamber 54. This volume may be determined by monitoring the weights of the plasma collection container 42 and/or the buffy coat or white blood cell layer collection container 44 (with a target change in weight corresponding to a change in fluid volume) or by monitoring the operational rate of the plasma pump 18. In another embodiment, the plasma maximization stage may continue until the optical sensor 34 detects a non-plasma fluid (e.g., the buffy coat or white blood cell layer) flowing through line L3.

After the plasma maximization stage has been completed (or after the collection stage has been completed, if the plasma maximization stage is not implemented), the optional red blood cell recovery stage (Fig. 9) may begin. During the red blood cell recovery stage, air from the plasma collection container 42 (which was conveyed there when priming the fluid flow circuit 12) is used to recover separated red blood cells remaining in the processing chamber 54 without removing the buffy coat or white blood cell layer from the processing chamber 54.

In the illustrated embodiment, the whole blood pump 16 is deactivated (if not deactivated as part of the plasma maximization stage) and the additive pump 20 is reactivated (if deactivated as part of the plasma maximization stage), while the plasma pump 18 is operated in a reverse direction (with respect to its direction of operation up to this stage of the procedure). This draws the air from the plasma collection container 42 and into line L7. Valve 38a is closed, while clamp 24c is open, which directs the air through line L7, into and through line L3, and into the processing chamber 54 via the plasma outlet port. On account of the air flowing through the plasma outlet port, it will enter the processing chamber 54 at the low-g side. As additional air is introduced into the processing chamber 54, it will move from the low-g wall towards the high-g wall, thus displacing the separated red blood cells in the processing chamber 54 through the red blood cell outlet port at the high-g side and into line L4. During this stage, the centrifuge 22 may be operated at a slower rate (e.g., in the range of approximately 1,000-2,000 rpm) to decrease the risk of an air blockage.

The additive pump 20 operates to draw additive solution from the additive solution container 48 and through line L10, to be mixed with the red blood cells flowing through line L4 at the junction of the two lines L4 and L10. The mixture continues flowing into and through line L5. If, at the end of the collection stage, the valve system was arranged so as to direct flow through the leukoreduction filter 64, valves 38a, 38b, and 38c may remain closed, with valve 38d being open to direct the mixture into line L11 for leukoreduction, as in Fig. 9. On the other hand, if the valve system was arranged so as to bypass the leukoreduction filter 64, valves 38a, 38c, and 38d may remain closed, with valve 38b being open to direct the mixture through lines L8 and L13 to bypass the leukoreduction filter 64. As described above with regard to the collection stage, it is possible for the controller to change the configurations of the valve system during the red blood cell recovery stage to stop leukoreduction of the mixture (e.g., if the pressure of the leukoreduction filter 64 becomes too great).

Regardless of whether the mixture is filtered, it flows into line L12, through open clamp 24a, and into the red blood cell collection container 46 (as a second portion of the collected red blood cells). The red blood cell recovery stage continues until the red blood cells have been removed from the processing chamber 54. This may be determined in any of a number of ways without departing from the scope of the present disclosure. In one embodiment, the red blood cell recovery stage continues until a predetermined volume of fluid (corresponding to the volume of red blood cells remaining in the processing chamber 54) has been conveyed out of the processing chamber 54. This volume may be calculated for example, by determining the volume of red blood cells present in the whole blood that has been processed (which may be determined based on the hematocrit of the blood) and then subtracting the volume of red blood cells that have already been conveyed into the red blood cell collection container 46 (which may be determined based on the weights of the red blood cell container and the additive solution container 48 at the end of the collection stage). In another embodiment, the red blood cell recovery stage may continue until the optical sensor 34 detects a non-red blood cell fluid (e.g., the buffy coat or white blood cell layer) flowing through line L4.

After the red blood cell recovery stage has been completed (or immediately following the collection stage or the plasma maximization stage, depending on the procedure), the buffy coat harvest stage (Fig. 10) will begin. During this stage, air and/or plasma from the plasma collection container 42 is used to convey the buffy coat or white blood cell layer in the processing chamber 54 into the buffy coat or white blood cell layer collection container 44 as a buffy coat or white blood cell product. The flow path of fluid through the fluid flow circuit 12 is the same (as shown in Fig. 10) regardless of whether air or plasma or both are used to collect the buffy coat or white blood cell product, though it may be advantageous for the orientation of the plasma collection container 42 to be dependent upon which fluid is used. In particular, when the plasma collection container 42 is oriented such that line L7 is associated with a top edge of the plasma collection container 42 (as in the illustrated embodiment), air will be drawn from the plasma collection container 42 before plasma (on account of the plasma in the container 42 being spaced away from line L7 at the top of the container 42). On the other hand, when the plasma collection container 42 is oriented such that line L7 is associated with a bottom edge of the plasma collection container 42, plasma will be drawn from the plasma collection container 42 before air (on account of the plasma in the container 42 being located adjacent to line L7 at the bottom of the container 42). Thus, an operator may orient the plasma collection container 42 or select a fluid flow circuit 12 having a particularly configured plasma collection container 42 based on whether air and/or plasma is to be used to collect the buffy coat or white blood cell product.

While either air or plasma may be used to harvest the buffy coat or white blood cell layer, it may be advantageous for various reasons to use plasma instead of air. For example, harvesting the buffy coat or white blood cell layer with plasma may be advantageous for preserving the quality of any red blood cells in the buffy coat or white blood cell layer. This is because, when harvesting with air, there is an increased risk of red blood cell hemolysis due to high shear that occurs between an air/red blood cell fluid interface within the processing chamber 54 and fluid lines, which is not present between a plasma/red blood cell interface. As a result, it may be necessary to operate the plasma pump 18 at a slower rate when harvesting with air compared to plasma, which increases procedure time. Nonetheless, if air is used to harvest all or a portion of the buffy coat or white blood cell layer, it may be advantageous for the centrifuge 22 to operate at a relatively low rate (e.g., in the range of approximately 1,000-2,000 rpm, as during the red blood cell recovery stage) to decrease the risk of an air blockage.

During the buffy coat harvest stage, clamps 24b and 24c are opened, while clamp 24a is closed. If either of valves 38b and 38d is open (which will be the case at the end of the red blood cell recovery stage, if it is executed), that valve will be closed during the buffy coat harvest stage. The whole blood pump 16 and the additive pump 20 (if currently operating) are also stopped upon transitioning to the buffy coat harvest stage.

With the various clamps, valves, and pumps being configured as shown in Fig. 10, the plasma pump 18 will drawing air or plasma from the plasma collection container 42 into the processing chamber 54 via lines L7 and L3. As in the red blood cell recovery stage, the air or plasma will enter the processing chamber 54 at the low-g side and move from the low-g wall toward the high-g wall as additional air or plasma is conveyed into the processing chamber 54. This displaces the buffy coat or white blood cell layer remaining in the processing chamber 54 through the red blood cell outlet port at the high-g side and into line L4. The buffy coat or white blood cell layer is directed from line L4 into line L5 and then into line L6. From line L6, the buffy coat or white blood cell layer is directed into line L16, through open clamp 24b, and into the buffy coat or white blood cell layer collection container 44 as a buffy coat or white blood cell product.

The buffy coat harvest stage continues until the buffy coat or white blood cell layer has been removed from the processing chamber 54. This may be determined in any of a number of ways without departing from the scope of the present disclosure. In one embodiment, the buffy coat harvest stage continues until a predetermined volume of fluid (corresponding to the volume of the buffy coat or white blood cell layer in the processing chamber 54) has been conveyed out of the processing chamber 54. This volume may be calculated for example, by subtracting the volumes of collected plasma and collected red blood cells at the end of the red blood cell recovery stage from the volume of blood that has been processed. In another embodiment, the buffy coat harvest stage may continue until the optical sensor 34 detects a non-buffy coat or white blood cell layer fluid (e.g., air or plasma) flowing through line L4.

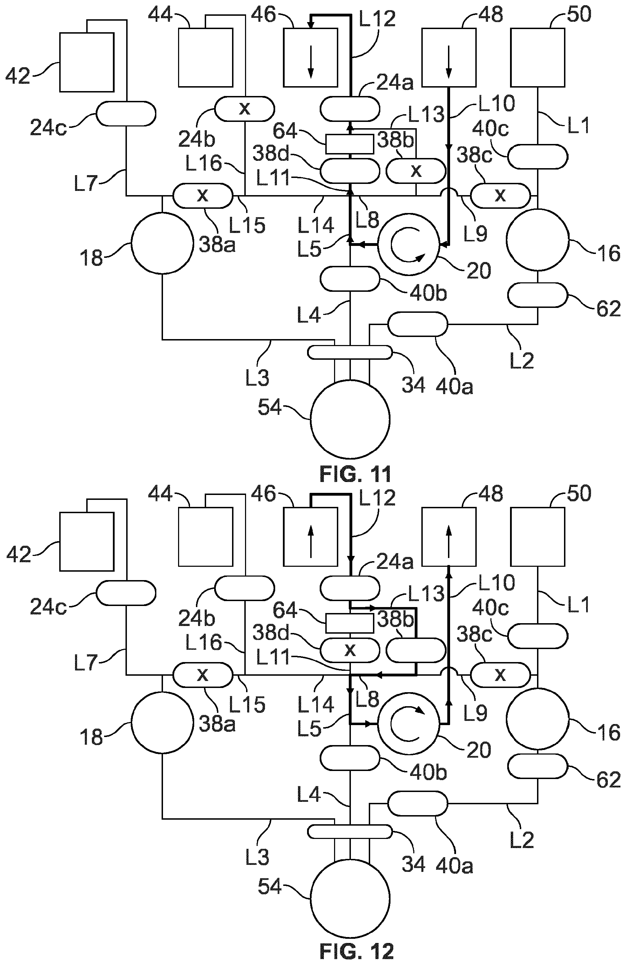

Once the buffy coat harvest stage is complete, the procedure may transition to an optional "additive solution flush" stage (Fig. 11). During the additive solution flush stage, additive solution from the additive solution container 48 is conveyed into the red blood cell collection container 46 until a target amount of additive solution is in the red blood cell collection container 46.

To transition from the buffy coat harvest stage to the additive solution flush stage, clamp 24b is closed, clamp 24a is opened, the plasma pump 18 is deactivated, and the additive pump 20 is reactivated. Additionally, one of valves 38b and 38d is opened, which determines whether the additive solution will be conveyed through the leukoreduction filter 64 (as shown in Fig. 11) or routed around the leukoreduction filter 64 (via line L13) and which may depend on which of the two valves 38b and 38d was open at the end of the red blood cell recovery stage. Typically, if valve 38d was open at the end of the red blood cell recovery stage (as in Fig. 9), it will be opened at the beginning of the additive solution flush stage (as in Fig. 11) to allow for red blood cells remaining in the leukoreduction filter 64 to be recovered by additive solution being conveyed through the leukoreduction filter 64. Similarly, if valve 38b was open at the end of the red blood cell recovery stage, it will be opened at the beginning of the additive solution flush stage to direct additive solution around the leukoreduction filter 64. However, it is also within the scope of the present disclosure for valve 38b to be closed and valve 38d to be open at the end of the red blood cell recovery stage (as in Fig. 9), with valve 38b being open and valve 38d being closed at the beginning of the additive solution flush stage, if the controller determines that it is advisable to begin bypassing the leukoreduction filter 64. Additionally, it is within the scope of the present disclosure for the valve system to be arranged as in Fig. 11 at the beginning of the additive solution flush stage (to direct additive solution through the leukoreduction filter 64) and to transition into a bypass configuration before the end of the additive solution flush stage.

The additive solution flush stage will continue until a target amount of additive solution has been added to the red blood cell collection container 46. When the additive solution flush stage is complete, the system may transition to an optional "air evacuation" stage, as shown in Fig. 12. During the air evacuation stage, the red blood cell collection container 46 is "burped" to remove all residual air for storage (just as air may be removed from the plasma collection container 42 during the red blood cell recovery and/or buffy coat harvest stages). This is done by reversing the direction of operation of the additive pump 20, closing valve 38d (if not already closed at the end of the additive solution flush stage), and opening valve 38b (if not already open at the end of the additive solution flush stage). The additive pump 20 draws air out of the red blood cell collection container 46 and into the additive solution container 48. While Fig. 12 shows the air being evacuated from the red blood cell collection container 46 to the additive solution container 48, it is within the scope of the present disclosure for all or a portion of the air to be directed to a different location of the fluid flow circuit 12.

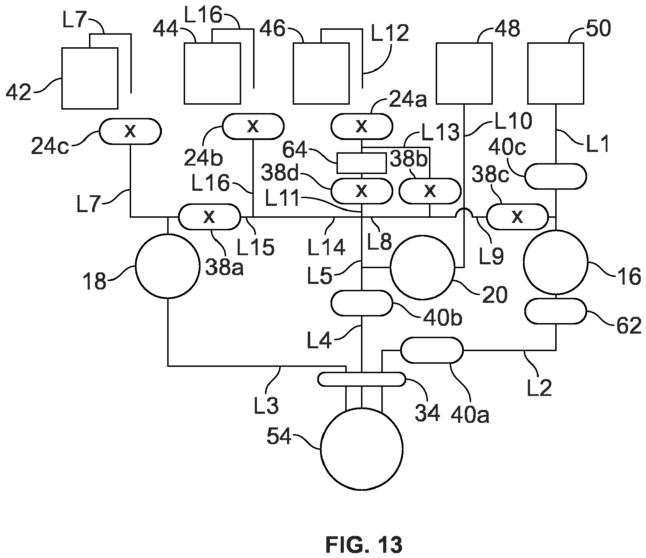

The air evacuation stage will continue until all of the air is removed from the red blood cell collection container 46. Upon completion of the air evacuation stage, any of a number of post-processing stages may be executed. For example, Fig. 13 shows a "sealing" stage in which all of the clamps and valves are closed and all of the pumps are deactivated. The line L12 connected to the red blood cell collection container 46, the line L7 connected to the plasma collection container 42, and the line L16 connected to the buffy coat or white blood cell layer collection container 44 are sealed and optionally severed for storage of the plasma, red blood cell, and buffy coat or white blood cell products. Optionally, air may be removed from the buffy coat or white blood cell layer collection container 44 (e.g., using a variation of the air evacuation stage of Fig. 12) before sealing line L16, though doing so is typically not necessary, especially for collected buffy coat, as it is common for a collected buffy coat to be pooled with other buffy coats, with the pooled buffy coats being processed to create a platelet product.

It should be understood that the processing device 10 shown in Fig. 1, the fluid flow circuit 12 shown in Figs. 2 and 3, and the procedure shown in Figs. 4-13 are merely exemplary of a system and procedure that may employ the techniques and principles described herein. Indeed, differently configured blood separation systems and separation procedures including different steps may employ the techniques and principles described herein without departing from the scope of the present disclosure.

### Aspects

Aspect 1. A blood processing device, comprising: a pump system; a centrifuge; and a controller configured to actuate the pump system to convey blood from a blood source into the centrifuge, actuate the centrifuge to separate the blood in the centrifuge into plasma, red blood cells, and a buffy coat or white blood cell layer between the separated plasma and the separated red blood cells, actuate the pump system to convey the separated plasma and the separated red blood cells out of the centrifuge for collection, and actuate the pump system to convey a portion of the collected plasma into the centrifuge to convey the buffy coat or white blood cell layer out of the centrifuge for collection.

Aspect 2. The blood processing device of Aspect 1, wherein the controller is configured to continue actuating the pump system to convey said portion of the collected plasma into the centrifuge until a predetermined volume of fluid has been displaced from the centrifuge.

Aspect 3. The blood processing device of Aspect 1, further comprising an optical sensor configured to monitor fluid exiting the centrifuge, wherein the controller is configured to continue actuating the pump system to convey said portion of the collected plasma into the centrifuge until the optical sensor detects a non-buffy coat or white blood cell layer fluid exiting the centrifuge.

Aspect 4. The blood processing device of any one of the preceding Aspects, wherein the controller is configured to actuate the pump system to simultaneously convey a first portion of the separated red blood cells out of the centrifuge and into a buffy coat or white blood cell layer collection container, and convey a first portion of the separated plasma out of the centrifuge and into a plasma collection container.

Aspect 5. The blood processing device of Aspect 4, wherein the controller is configured to actuate the pump system to simultaneously convey a second portion of the separated red blood cells out of the centrifuge and into a red blood cell collection container, and convey a second portion of the separated plasma out of the centrifuge and into the plasma collection container.

Aspect 6. The blood processing device of any one of Aspects 4-5, wherein the controller is configured to actuate the pump system to convey said first portion of the separated red blood cells out of the buffy coat or white blood cell layer collection container and into the centrifuge so as to convey a third portion of the separated plasma into the plasma collection container.

Aspect 7. The blood processing device of any one of Aspects 4-6, wherein the blood processing device is configured for use in combination with a fluid flow circuit including the plasma collection container, the controller is configured to actuate the pump system to convey air in the fluid flow circuit into the plasma collection container prior to actuating the centrifuge to separate the blood in the centrifuge, the controller is configured to actuate the pump system to convey at least a portion of the air in the plasma collection container into the centrifuge to convey a third portion of the separated red blood cells out of the centrifuge for collection, and the controller is configured to actuate the pump system to convey said portion of the collected plasma from the plasma collection container into the centrifuge to convey the buffy coat or white blood cell layer out of the centrifuge for collection in the buffy coat or white blood cell layer collection container after collecting said third portion of the separated red blood cells.

Aspect 8. A blood processing system, comprising: a reusable processing device including a pump system, a centrifuge, and a controller; and a disposable fluid flow circuit including a processing chamber received by the centrifuge, a buffy coat or white blood cell layer collection container, and a plurality of conduits fluidly connecting the components of the fluid flow circuit, wherein the controller is configured to actuate the pump system to convey blood from a blood source into the processing chamber, actuate the centrifuge to separate the blood in the processing chamber into plasma, red blood cells, and a buffy coat or white blood cell layer between the separated plasma and the separated red blood cells, actuate the pump system to convey the separated plasma and the separated red blood cells out of the processing chamber for collection, and actuate the pump system to convey a portion of the collected plasma into the processing chamber to convey the buffy coat or white blood cell layer out of the processing chamber and into the buffy coat or white blood cell collection container.

Aspect 9. The blood processing system of Aspect 8, wherein the controller is configured to continue actuating the pump system to convey said portion of the collected plasma into the processing chamber until a predetermined volume of fluid has been displaced from the processing chamber.

Aspect 10. The blood processing system of Aspect 8, wherein the processing device includes an optical sensor configured to monitor fluid exiting the processing chamber, and the controller is configured to continue actuating the pump system to convey said portion of the collected plasma into the processing chamber until the optical sensor detects a non-buffy coat or white blood cell layer fluid exiting the processing chamber.

Aspect 11. The blood processing system of any one of Aspects 8-10, wherein the controller is configured to actuate the pump system to simultaneously convey a first portion of the separated red blood cells out of the processing chamber and into the buffy coat or white blood cell layer collection container, and convey a first portion of the separated plasma out of the processing chamber and into a plasma collection container of the fluid flow circuit.

Aspect 12. The blood processing system of Aspect 11, wherein the controller is configured to actuate the pump system to simultaneously convey a second portion of the separated red blood cells out of the processing chamber and into a red blood cell collection container of the fluid flow circuit, and convey a second portion of the separated plasma out of the processing chamber and into the plasma collection container.

Aspect 13. The blood processing system of any one of Aspects 11-12, wherein the controller is configured to actuate the pump system to convey said first portion of the separated red blood cells out of the buffy coat or white blood cell layer collection container and into the processing chamber so as to convey a third portion of the separated plasma into the plasma collection container.

Aspect 14. The blood processing system of any one of Aspect 11-13, wherein the controller is configured to actuate the pump system to convey air in the fluid flow circuit into the plasma collection container prior to actuating the centrifuge to separate the blood in the processing chamber, the controller is configured to actuate the pump system to convey at least a portion of the air in the plasma collection container into the processing chamber to convey a third portion of the separated red blood cells out of the processing chamber for collection, and the controller is configured to actuate the pump system to convey said portion of the collected plasma from the plasma collection container into the processing chamber to convey the buffy coat or white blood cell layer out of the processing chamber for collection in the buffy coat or white blood cell layer collection container after collecting said third portion of the separated red blood cells.

Aspect 15. A method for collecting a buffy coat or white blood cell product, comprising: conveying blood from a blood source into a centrifuge; separating the blood in the centrifuge into plasma, red blood cells, and a buffy coat or white blood cell layer between the separated plasma and the separated red blood cells; conveying the separated plasma and the separated red blood cells out of the centrifuge for collection; and conveying a portion of the collected plasma into the centrifuge to convey the buffy coat or white blood cell layer out of the centrifuge for collection.

Aspect 16. The method of Aspect 15, wherein said conveying said portion of the collected plasma into the centrifuge includes conveying said portion of the collected plasma into the centrifuge until a predetermined volume of fluid has been displaced from the centrifuge.

Aspect 17. The method of Aspect 15, wherein said conveying said portion of the collected plasma into the centrifuge includes conveying said portion of the collected plasma into the centrifuge until a non-buffy coat or white blood cell layer fluid is detected exiting the centrifuge.

Aspect 18. The method of any one of Aspects 15-17, wherein said conveying the separated plasma and the separated red blood cells out of the centrifuge for collection includes simultaneously conveying a first portion of the separated red blood cells out of the centrifuge and into a buffy coat or white blood cell layer collection container, and conveying a first portion of the separated plasma out of the centrifuge and into a plasma collection container.

Aspect 19. The method of Aspect 18, wherein said conveying the separated plasma and the separated red blood cells out of the centrifuge for collection includes simultaneously conveying a second portion of the separated red blood cells out of the centrifuge and into a red blood cell collection container, and conveying a second portion of the separated plasma out of the centrifuge and into the plasma collection container.

Aspect 20. The method of any one of Aspects 18-19, further comprising conveying said first portion of the separated red blood cells out of the buffy coat or white blood cell layer collection container and into the centrifuge so as to convey a third portion of the separated plasma into the plasma collection container.

Aspect 21. The method of any one of Aspects 18-20, further comprising conveying air into the plasma collection container prior to separating the blood in the centrifuge, conveying at least a portion of the air in the plasma collection container into the centrifuge to convey a third portion of the separated red blood cells out of the centrifuge for collection, and conveying said portion of the collected plasma from the plasma collection container into the centrifuge to convey the buffy coat or white blood cell layer out of the centrifuge for collection in the buffy coat or white blood cell layer collection container after collecting said third portion of the separated red blood cells.

Aspect 22. A blood processing device, comprising: a pump system; a centrifuge; and a controller configured to actuate the pump system to convey blood from a blood source into the centrifuge, actuate the centrifuge to separate the blood in the centrifuge into separated plasma and separated red blood cells, actuate the pump system to simultaneously convey a first portion of the separated plasma out the centrifuge and into a plasma collection container, and convey a first portion of the separated red blood cells out of the centrifuge and into a temporary storage container, and actuate the pump system to convey said first portion of the separated red blood cells out of the temporary storage container and into the centrifuge to convey an additional portion of the separated plasma into the plasma collection container.

Aspect 23. The blood processing device of Aspect 22, wherein the controller is configured to actuate the pump system to simultaneously convey a second portion of the separated red blood cells out of the centrifuge and into a red blood cell collection container, and convey a second portion of the separated plasma out of the centrifuge and into the plasma collection container.

Aspect 24. The blood processing device of Aspect 23, wherein the controller is configured to actuate the pump system to convey said first portion of the separated red blood cells out of the temporary storage container and into the centrifuge to convey said additional portion of the separated plasma into the plasma collection container after actuating the pump system to convey said second portions of the separated red blood cells and the separated plasma out of the centrifuge.

Aspect 25. The blood processing device of any one of Aspects 23-24, wherein the blood processing device is configured for use in combination with a fluid flow circuit including the plasma collection container, the controller is configured to actuate the pump system to convey air in the fluid flow circuit into the plasma collection container prior to actuating the centrifuge to separate the blood in the centrifuge, and the controller is configured to actuate the pump system to convey at least a portion of the air in the plasma collection container into the centrifuge to convey a third portion of the separated red blood cells out of the centrifuge for collection.

Aspect 26. The blood processing device of any one of Aspects 22-25, further comprising a weight scale configured to weigh the separated red blood cells in the temporary storage container or the separated plasma in the plasma collection container, wherein the controller is configured to continue actuating the pump system to convey said first portion of the separated red blood cells out of the temporary storage container and into the centrifuge until the weight scale has measured a target change in weight.

Aspect 27. The blood processing device of any one of Aspects 22-25, wherein the controller is configured to continue actuating the pump system to convey said first portion of the separated red blood cells out of the temporary storage container and into the centrifuge until a predetermined volume of fluid has been displaced from the centrifuge.

Aspect 28. The blood processing device of any one of Aspects 22-25, further comprising an optical sensor configured to monitor fluid exiting the centrifuge, wherein the controller is configured to continue actuating the pump system to convey said first portion of the separated red blood cells out of the temporary storage container and into the centrifuge until the optical sensor detects a non-plasma fluid exiting the centrifuge.

Aspect 29. A blood processing system, comprising: a reusable processing device including a pump system, a centrifuge, and a controller; and a disposable fluid flow circuit including a processing chamber received by the centrifuge, a temporary storage container, a plasma collection container, and a plurality of conduits fluidly connecting the components of the fluid flow circuit, wherein the controller is configured to actuate the pump system to convey blood from a blood source into the processing chamber, actuate the centrifuge to separate the blood in the processing chamber into separated plasma and separated red blood cells, actuate the pump system to simultaneously convey a first portion of the separated plasma out the processing chamber and into the plasma collection container, and convey a first portion of the separated red blood cells out of the centrifuge and into the temporary storage container, and actuate the pump system to convey said first portion of the separated red blood cells out of the temporary storage container and into the processing chamber to convey an additional portion of the separated plasma into the plasma collection container.

Aspect 30. The blood processing system of Aspect 29, wherein the controller is configured to actuate the pump system to simultaneously convey a second portion of the separated red blood cells out of the processing chamber and into a red blood cell collection container of the fluid flow circuit, and convey a second portion of the separated plasma out of the processing chamber and into the plasma collection container.

Aspect 31. The blood processing system of Aspect 30, wherein the controller is configured to actuate the pump system to convey said first portion of the separated red blood cells out of the temporary storage container and into the processing chamber to convey said additional portion of the separated plasma into the plasma collection container after actuating the pump system to convey said second portions of the separated red blood cells and the separated plasma out of the processing chamber.

Aspect 32. The blood processing system of any one of Aspects 30-31, wherein the controller is configured to actuate the pump system to convey air in the fluid flow circuit into the plasma collection container prior to actuating the centrifuge to separate the blood in the processing chamber, and the controller is configured to actuate the pump system to convey at least a portion of the air in the plasma collection container into the processing chamber to convey a third portion of the separated red blood cells out of the processing chamber for collection.

Aspect 33. The blood processing system of any one of Aspects 29-32, further comprising a weight scale configured to weigh the separated red blood cells in the temporary storage container or the separated plasma in the plasma collection container, wherein the controller is configured to continue actuating the pump system to convey said first portion of the separated red blood cells out of the temporary storage container and into the processing chamber until the weight scale has measured a target change in weight.

Aspect 34. The blood processing system of any one of Aspects 29-32, wherein the controller is configured to continue actuating the pump system to convey said first portion of the separated red blood cells out of the temporary storage container and into the processing chamber until a predetermined volume of fluid has been displaced from the processing chamber.

Aspect 35. The blood processing system of any one of Aspects 29-32, further comprising an optical sensor configured to monitor fluid exiting the processing chamber, wherein the controller is configured to continue actuating the pump system to convey said first portion of the separated red blood cells out of the temporary storage container and into the processing chamber until the optical sensor detects a non-plasma fluid exiting the processing chamber.

Aspect 36. A method for collecting plasma, comprising: conveying blood from a blood source into a centrifuge; separating the blood in the centrifuge into separated plasma and separated red blood cells; simultaneously conveying a first portion of the separated plasma out the centrifuge and into a plasma collection container, and a first portion of the separated red blood cells out of the centrifuge and into a temporary storage container; and conveying said first portion of the separated red blood cells out of the temporary storage container and into the centrifuge to convey an additional portion of the separated plasma into the plasma collection container.

Aspect 37. The method of Aspect 36, further comprising simultaneously conveying a second portion of the separated red blood cells out of the centrifuge and into a red blood cell collection container, and a second portion of the separated plasma out of the centrifuge and into the plasma collection container.

Aspect 38. The method of Aspect 37, wherein said first portion of the separated red blood cells is conveyed out of the temporary storage container and into the centrifuge to convey said additional portion of the separated plasma into the plasma collection container after said second portions of the separated red blood cells and the separated plasma are conveyed out of the centrifuge.

Aspect 39. The method of any one of Aspects 37-38, further comprising conveying air into the plasma collection container prior to separating the blood in the centrifuge, and conveying at least a portion of the air in the plasma collection container into the centrifuge to convey a third portion of the separated red blood cells out of the centrifuge for collection.

Aspect 40. The method of any one of Aspects 36-39, further comprising measuring the weight of the separated red blood cells in the temporary storage container or the weight of the separated plasma in the plasma collection container, wherein said conveying said first portion of the separated red blood cells out of the temporary storage container and into the centrifuge continues until measuring a target change in weight.

Aspect 41. The method of any one of Aspects 36-39, wherein said conveying said first portion of the separated red blood cells out of the temporary storage container and into the processing chamber continues until a predetermined volume of fluid has been displaced from the centrifuge.

Aspect 42. The method of any one of Aspects 36-39, further comprising optically monitoring fluid exiting the centrifuge, wherein said conveying said first portion of the separated red blood cells out of the temporary storage container and into the centrifuge continues until optically detecting a non-plasma fluid exiting the centrifuge.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood processing device (10), comprising:
a pump system (16, 18, 20);
a centrifuge (22); and
a controller configured to
actuate the pump system (16, 18, 20) to convey blood from a blood source (50) into the centrifuge (22),
actuate the centrifuge (22) to separate the blood in the centrifuge (22) into plasma, red blood cells, and a buffy coat or white blood cell layer between the separated plasma and the separated red blood cells,
actuate the pump system (16, 18, 20) to convey the separated plasma and the separated red blood cells out of the centrifuge (22) for collection, and
actuate the pump system (16, 18, 20) to convey a portion of the collected plasma into the centrifuge (22) to convey the buffy coat or white blood cell layer out of the centrifuge (22) for collection.

2. The blood processing device (10) of claim 1, wherein the controller is configured to continue actuating the pump system (16, 18, 20) to convey said portion of the collected plasma into the centrifuge (22) until a predetermined volume of fluid has been displaced from the centrifuge (22).

3. The blood processing device (10) of claim 1, further comprising an optical sensor (34) configured to monitor fluid exiting the centrifuge (22), wherein the controller is configured to continue actuating the pump system (16, 18, 20) to convey said portion of the collected plasma into the centrifuge (22) until the optical sensor (34) detects a non-buffy coat or white blood cell layer fluid exiting the centrifuge (22).

4. The blood processing device (10) of any one of the preceding claims, wherein the controller is configured to actuate the pump system (16, 18, 20) to simultaneously
convey a first portion of the separated red blood cells out of the centrifuge (22) and into a buffy coat or white blood cell layer collection container (44), and
convey a first portion of the separated plasma out of the centrifuge (22) and into a plasma collection container (42).

5. The blood processing device (10) of claim 4, wherein the controller is configured to actuate the pump system (16, 18, 20) to simultaneously
convey a second portion of the separated red blood cells out of the centrifuge (22) and into a red blood cell collection container (46), and
convey a second portion of the separated plasma out of the centrifuge (22) and into the plasma collection container (42).

6. The blood processing device (10) of any one of claims 4-5, wherein the controller is configured to actuate the pump system (16, 18, 20) to convey said first portion of the separated red blood cells out of the buffy coat or white blood cell layer collection container (44) and into the centrifuge (22) so as to convey a third portion of the separated plasma into the plasma collection container (42).

7. The blood processing device (10) of any one of claims 4-6, wherein
the blood processing device (10) is configured for use in combination with a fluid flow circuit (12) including the plasma collection container (42),
the controller is configured to actuate the pump system (16, 18, 20) to convey air in the fluid flow circuit (12) into the plasma collection container (42) prior to actuating the centrifuge (22) to separate the blood in the centrifuge (22),
the controller is configured to actuate the pump system (16, 18, 20) to convey at least a portion of the air in the plasma collection container (42) into the centrifuge (22) to convey a third portion of the separated red blood cells out of the centrifuge (22) for collection, and
the controller is configured to actuate the pump system (16, 18, 20) to convey said portion of the collected plasma from the plasma collection container (42) into the centrifuge (22) to convey the buffy coat or white blood cell layer out of the centrifuge (22) for collection in the buffy coat or white blood cell layer collection container (44) after collecting said third portion of the separated red blood cells.

8. A blood processing system, comprising:
the blood processing device (10) of any one of the preceding claims; and
a disposable fluid flow circuit (12) including a processing chamber (54) received by the centrifuge (22), a buffy coat or white blood cell layer collection container (44), and a plurality of conduits fluidly connecting the components of the fluid flow circuit (12), wherein the controller is configured to
actuate the pump system (16, 18, 20) to convey blood from the blood source (50) into the processing chamber (54),
actuate the centrifuge (22) to separate the blood in the processing chamber (54) into plasma, red blood cells, and the buffy coat or white blood cell layer between the separated plasma and the separated red blood cells,
actuate the pump system (16, 18, 20) to convey the separated plasma and the separated red blood cells out of the processing chamber (54) for collection, and
actuate the pump system (16, 18, 20) to convey said portion of the collected plasma into the processing chamber (54) to convey the buffy coat or white blood cell layer out of the processing chamber (54) and into the buffy coat or white blood cell collection container (44).

9. A blood processing device (10), comprising:
a pump system (16, 18, 20);
a centrifuge (22); and
a controller configured to
actuate the pump system (16, 18, 20) to convey blood from a blood source (50) into the centrifuge (22),
actuate the centrifuge (22) to separate the blood in the centrifuge (22) into separated plasma and separated red blood cells,
actuate the pump system (16, 18, 20) to simultaneously
convey a first portion of the separated plasma out the centrifuge (22) and into a plasma collection container (42), and
convey a first portion of the separated red blood cells out of the centrifuge (22) and into a temporary storage container (44), and
actuate the pump system (16, 18, 20) to convey said first portion of the separated red blood cells out of the temporary storage container (44) and into the centrifuge (22) to convey an additional portion of the separated plasma into the plasma collection container (42).

10. The blood processing device (10) of claim 9, wherein the controller is configured to actuate the pump system (16, 18, 20) to simultaneously
convey a second portion of the separated red blood cells out of the centrifuge (22) and into a red blood cell collection container (46), and
convey a second portion of the separated plasma out of the centrifuge (22) and into the plasma collection container (42).

11. The blood processing device (10) of claim 10, wherein the controller is configured to actuate the pump system (16, 18, 20) to convey said first portion of the separated red blood cells out of the temporary storage container (44) and into the centrifuge (22) to convey said additional portion of the separated plasma into the plasma collection container (42) after actuating the pump system (16, 18, 20) to convey said second portions of the separated red blood cells and the separated plasma out of the centrifuge (22).

12. The blood processing device (10) of any one of claims 10-11, wherein
the blood processing device (10) is configured for use in combination with a fluid flow circuit (12) including the plasma collection container (42),
the controller is configured to actuate the pump system (16, 18, 20) to convey air in the fluid flow circuit (12) into the plasma collection container (42) prior to actuating the centrifuge (22) to separate the blood in the centrifuge (22), and
the controller is configured to actuate the pump system (16, 18, 20) to convey at least a portion of the air in the plasma collection container (42) into the centrifuge (22) to convey a third portion of the separated red blood cells out of the centrifuge (22) for collection.

13. The blood processing device (10) of any one of claims 9-12, further comprising a weight scale configured to weigh the separated red blood cells in the temporary storage container (44) or the separated plasma in the plasma collection container (42), wherein the controller is configured to continue actuating the pump system (16, 18, 20) to convey said first portion of the separated red blood cells out of the temporary storage container (44) and into the centrifuge (22) until the weight scale has measured a target change in weight.

14. The blood processing device (10) of any one of claims 9-12, wherein the controller is configured to continue actuating the pump system (16, 18, 20) to convey said first portion of the separated red blood cells out of the temporary storage container (44) and into the centrifuge (22) until a predetermined volume of fluid has been displaced from the centrifuge (22).

15. The blood processing device (10) of any one of claims 9-12, further comprising an optical sensor (34) configured to monitor fluid exiting the centrifuge (22), wherein the controller is configured to continue actuating the pump system (16, 18, 20) to convey said first portion of the separated red blood cells out of the temporary storage container (44) and into the centrifuge (22) until the optical sensor (34) detects a non-plasma fluid exiting the centrifuge (22).

16. A blood processing system, comprising:
the blood processing device (10) of any one of claims 9-15; and
a disposable fluid flow circuit (12) including a processing chamber (54) received by the centrifuge (22), a temporary storage container (44), a plasma collection container (42), and a plurality of conduits fluidly connecting the components of the fluid flow circuit (12), wherein the controller is configured to
actuate the pump system (16, 18, 20) to convey blood from the blood source (50) into the processing chamber (54),
actuate the centrifuge (22) to separate the blood in the processing chamber (54) into separated plasma and separated red blood cells,
actuate the pump system (16, 18, 20) to simultaneously
convey said first portion of the separated plasma out the processing chamber (54) and into the plasma collection container (42), and
convey said first portion of the separated red blood cells out of the centrifuge (22) and into the temporary storage container (44), and
actuate the pump system (16, 18, 20) to convey said first portion of the separated red blood cells out of the temporary storage container (44) and into the processing chamber (54) to convey said additional portion of the separated plasma into the plasma collection container (42).
